# FASCICULE DE BREVET EUROPEEN

(11) **EP 3 714 933 B1**
(45) Date de publication et mention de la délivrance du brevet: **21.07.2021**
(21) Numéro de dépôt: 20156231.1
(22) Date de dépôt: 07.02.2020
(51) Int. Cl.: A61M 25/02, A61M 25/00

(54) **CATHÉTER DONT LA FACE EXTERNE EST EN CARBONE**
KATHETER, DESSEN AUSSENSEITE AUS KOHLENSTOFF BESTEHT
CATHETER IN WHICH THE OUTER SURFACE IS MADE OF CARBON

(30) Priorité: 10.12.2018 FR 1872572
(43) Date de publication de la demande: 30.09.2020
(73) Titulaire: Zinzindohoue-Marsaudon, Franck, 07130 Saint-Peray (FR)
(72) Inventeur: Zinzindohoue-Marsaudon, Franck, 07130 Saint-Peray (FR)
(74) Mandataire: Gaglione, Renaud

(56) Documents cités:
- EP-A1- 1 731 191
- US-A- 5 984 896
- US-A- 6 007 544
- US-B1- 6 471 689

## Description

L'invention a trait au domaine des dispositifs médicaux. Elle concerne un cathéter insérable dans un organisme vivant (typiquement le corps humain) par voie percutanée, notamment pour l'administration d'un fluide tel qu'un soluté, une alimentation parentérale, un médicament intraveineux, des produits sanguins, un produit de contraste (par ex. via une seringue ou une tubulure de perfusion), pour des échanges sanguin comme lors d'une épuration extrarénale, ou pour l'introduction d'instruments filaires d'exploration (notamment par imagerie), d'instruments chirurgicaux (chirurgie mini-invasive), d'alimentation électrique ou de transmission de données informatiques ou se signaux électriques, ou encore pour prélever un fluide organique (par ex. sang, lymphe, épanchement, urines etc.). Ces cathéters peuvent être placés dans une veine, dans une artère, dans une cavité intracorporelle ou être un accès intracorporel dans le but d'un accès transcutané vers le milieu intérieur du corps.

Les cathéters sont connus de longue date ; leur usage en milieu médical est très fréquent : rien que pour les cathéters veineux périphériques, le rapport 2005 de la Haute Autorité de Santé sur la prévention des infections estime le nombre de leur mise en place à 25 millions/an. Selon le contexte jusqu'à 30% des patients hospitalisés sont porteur de ce type de dispositif d'après une étude réalisée par l'Institut de Veille Sanitaire (InVS, Enquête nationale de prévalence des infections nosocomiales et des traitements anti-infectieux en établissements de santé, mai-juin 2012).

Bien qu'il en existe de nombreux types, les cathéters ont généralement en commun de comprendre :
- A une extrémité proximale, un embout destiné à être positionné à l'extérieur de l'organisme pour une connexion externe ;
- Une canule, qui s'étend à partir de l'embout jusqu'à une extrémité distale destinée à être positionnée à l'intérieur de l'organisme.

La canule présente une face interne qui définit un conduit interne permettant le passage d'une aiguille de ponction, ou d'un autre objet filaire rigide ou non, ou encore d'un fluide. La canule présente également une face externe dont une partie est destinée à former l'interface du cathéter avec l'organisme.

Un cathéter typique est décrit dans le brevet français n°FR1567375.

La canule est généralement réalisée dans un polymère thermoplastique, comme le polyéthylène, le polypropylène (mentionnés dans le brevet précité). Le silicone, le polyuréthane, ou encore le polytétrafluoroéthylène (PTFE) sont également utilisés.

Quel que soit le matériau (connu) employé pour la réalisation de la canule, on observe fréquemment une colonisation microbienne (bactéries, champignons) de l'interface de la face externe avec l'organisme à partir du point de pénétration de la canule depuis la surface épithéliale. Cette colonisation microbienne induit un fort risque infectieux par propagation de bactéries ou de champignons vers l'intérieur de l'organisme. C'est une cause fréquente d'infection nosocomiale.

En milieu médical, le diagnostic de l'infection se fait ordinairement par détection visuelle d'une inflammation ou d'une suppuration au point de ponction, et, à un stade avancé, par des signes infectieux généraux (notamment fièvre) ainsi que des signes biologiques d'inflammation et d'infection.

Lorsqu'un cathéter est introduit dans une voie veineuse par exemple, la colonisation microbienne ne demeure pas localisée au point d'entrée mais passe dans le sang et, par la circulation sanguine, s'étend dans l'organisme au risque de provoquer une septicémie.

Dans la pratique, les tentatives de désinfection pour garder stérile le point d'entrée du cathéter se révèlent infructueuses après quelques jours, et il faut souvent changer le point de ponction, parfois procéder au retrait du cathéter en urgence, (et donc modifier l'acte médical en cours) et, selon la gravité de l'infection, administrer des anti- biotiques, allonger l'hospitalisation et donc augmenter les coûts en plus du risque vital.

Les cas d'infection nosocomiale induite par la colonisation microbienne des cathéters restent trop nombreux ; en outre, le taux de létalité des infections nosocomiales demeure élevé. C'est un problème majeur de santé publique.

Face à ces problèmes, les fabricants conçoivent ordinairement leurs cathéters implantables de sorte que leur retrait soit aussi aisé (et confortable pour le patient) que leur insertion. C'est pourquoi de nombreux cathéters ont une canule fabriquée dans un matériau à faible coefficient de frottement (notamment en PTFE, comme déjà évoqué) ou revêtue d'une couche superficielle dans un matériau tel, voire même d'un lubrifiant, à l'image des cathéters de la gamme Lubricath® commercialisée par la société Bard, l'objectif étant de favoriser le glissement de la canule contre les tissus organiques et diminuer la douleur lors de la ponction.

Sur la foi de ses observations cliniques, le personnel médical applique une doctrine d'usage des cathéters dont les règles peuvent s'énoncer ainsi :
- La pose du cathéter suit des règles d'hygiène strictes ;
- Un cathéter n'est pas un dispositif pérenne ;
- Un cathéter est le siège de proliférations microbiennes dont les conséquences peuvent être létales ;
- Le personnel médical doit surveiller étroitement et fréquemment l'apparition de toute inflammation ou suppuration ;
- La désinfection de l'interface à l'entrée du cathéter est impérative, et ce à fréquence au moins quotidienne ;
- Dans un délai relativement bref (quelques jours, quelques semaines tout au plus) le cathéter doit être retiré et remplacé par un nouveau si l'usage d'un cathéter est indispensable à l'acte médical en cours.

Ajoutons à ces problèmes le fait qu'en dépit de la biocompatibilité des matériaux employés, l'organisme ne cicatrise pas au point de ponction en présence du cathéter. Dans le meilleur des cas, les tissus développent une fibrose de réaction à corps étrangers autour de la canule. Cette fibrose rend d'ailleurs difficile la cicatrisation des tissus après le retrait du cathéter, et cette cicatrisation forme de fréquents et inesthétiques reliefs épidermiques.

Pour finir, on peut remarquer que la plupart des cathéters n'ont pas de moyen propre d'ancrage dans les tissus (et pour cause : il convient de pouvoir les retirer facilement et rapidement). Aussi, pour maintenir un tel cathéter en place, le personnel médical a-t-il couramment recours à des bandages ou des pansements qui augmentent la macération et risquent d'amplifier la prolifération bactérienne dans ce milieu clos, humide et chaud.

Quant aux cathéters qui sont pourvus de moyens d'ancrage (par ex. le cathéter de Hickman®) ou les cathéters dits centraux avec une base suturable, ils ne sont pas dénués d'inconvénients. Leur procédure de pose et de retrait est plus complexe (le cathéter est positionné au moyen d'un manchon sous-cutané ; un pansement doit être appliqué sur le site de ponction ou une suture chirurgicale est nécessaire) ; leur positionnement doit être contrôlé par radioscopie fréquemment lors de la pose. Les cas d'infection et d'hémorragie restent nombreux. Il faut une anesthésie locale pour l'incision et la suture lors de la pose et du retrait.

Le document US 6 471 689 divulgue un cathéter conforme au préambule de la revendication principale, dans lequel le manchon de protection des risques infectieux est réalisé dans un matériau à base de fibre chimique en polyester, connu encore sous le nom de dacron. Ce manchon est conçu dans ce matériau pour mieux ancrer le cathéter dans les tissus en favorisant la croissance cellulaire. En utilisation, le manchon occupe une position sous-cutanée.

Le document EP 1 731 191 divulgue un autre cathéter comprenant un manchon anti-infection, réalisé dans une résine thermodurcissable ou thermoplastique à structure poreuse pour favoriser la formation d'une interpénétration avec les tissus.

Le document US 5 984 896 divulgue un cathéter conforme au préambule de la revendication principale sauf en ce que le manchon est remplacé ici par un ruban réalisé dans un matériau métallique ou en carbone composite et disposé en spirale autour de la canule. De fait, il est présenté dans le document dans un but non d'anti-infection mais d'ancrage, raison pour laquelle sa surface externe possède un relief pour s'accrocher aux tissus. L'insertion du cathéter impose d'avoir recours à une aiguille creuse, pré-introduite dans les tissus pour faire passer la canule entourée par le ruban spiralé, puis de la retirer pour éviter au relief porté par la surface externe d'entraîner des blessures.

L'invention a pour objectif de remédier, autant que possible, aux problèmes qui viennent d'être énoncés, en proposant un cathéter qui, au lieu d'être insérable comme les cathéters connus, soit véritablement intégrable dans l'organisme, au sens où il s'intègre aux tissus de l'organisme en se plaçant en contact cellulaire intime avec eux. Il crée par son intégration une barrière à la prolifération infectieuse. Il est partiellement fixé par cette intégration.

À cet effet, il est proposé un cathéter insérable dans un organisme vivant par voie percutanée pour l'administration, le prélèvement ou l'échange de fluide ou l'introduction d'un instrument ou d'un objet filaire (par ex. un conducteur électrique), ce cathéter comprenant :
- A une extrémité proximale, un embout destiné à être positionné à l'extérieur de l'organisme (par ex vers une poche de fluide ou une source électrique) ;
- Une canule qui s'étend à partir de l'embout jusqu'à une extrémité distale destinée à être positionnée à l'intérieur de l'organisme, cette canule ayant une face interne définissant un conduit interne, et une face externe au contact des tissus depuis l'extérieur vers l'intérieur de l'organisme ;
- Un manchon réalisé dans un matériau qui compose ou qui recouvre tout ou partie de la face externe de la canule et qui est composé à 95% au moins de carbone, qu'il soit de type pyrolysé sous forme de matrice, sous forme pulvérisée, sous forme filaire ou sous forme composite filaire dans une matrice, qu'il soit de type polymérisé comme le carbone amorphe hydrogéné (par exemple le poly-hydridocarbyne) comme le Diamon-like carbone ou le polymer-like carbone, ou qu'il soit sous forme par association des types pyrolysés et polymérisés sous différentes de leurs formes.

Des tests conduits sur des organismes vivants ont montré que les tissus de l'épithélium (comprenant principalement l'épiderme et le derme et des muqueuses) non seulement ne développent pas de réaction à corps étranger vis-à-vis du carbone lorsque la surface de contact de l'organisme avec la canule, mais l'organisme intègre au contraire cette surface en carbone comme s'il s'agissait d'une partie de lui-même, les cellules de l'épithélium venant y adhérer sans interface de fibrose ni espace péri-prothétique. Il se produit même une sorte d'ancrage.

Diverses caractéristiques supplémentaires peuvent être prévues, seules ou en combinaison :
- Le manchon s'étend sur une partie ou toute la longueur de la canule.
- Le manchon peut constituer la totalité de la canule et non plus une couche externe.
- La canule présente, sur une partie de sa longueur, une restriction externe de calibre, dans laquelle s'étend le manchon de ou en carbone.
- Le manchon présente une face externe qui s'étend dans le prolongement de la face externe de la canule.
- La canule est surmoulée en partie dans le - et de part et d'autre du - manchon. Le manchon forme, par rapport à au moins une partie de la canule, une surépaisseur.
- La canule présente une section de moindre diamètre sur laquelle est déposé le manchon de carbone.
- Le manchon est effilé au moins à une extrémité située du côté de l'extrémité intracorporelle de la canule.
- La canule est réalisée dans un polymère thermoplastique.

D'autres objets et avantages de l'invention apparaîtront à la lumière de la description d'un mode de réalisation, faite ci-après en référence aux dessins annexés dans lesquels :
La FIG. 1 est une vue en perspective montrant un cathéter recouvert d'un manchon en carbone sous une forme pyrolysée, polymérisée, seule ou en as- sociation des deux selon un premier mode de réalisation pour l'administration de fluide.
La FIG. 2 est une vue en coupe longitudinale du cathéter de la FIG. 1 avec, en médaillon, des détails à plus grande échelle centrés, en haut, sur le manchon et, en bas, sur l'extrémité distale de la canule.
La FIG. 3 est une vue en coupe longitudinale partielle d'un cathéter pourvu d'un manchon en carbone sous une forme pyrolysée, polymérisée, seule ou en association dans une variante de réalisation avec, en médaillon, un détail à plus grande échelle centré sur le manchon.
La FIG. 4 est une vue en coupe transversale du cathéter, suivant le plan IV-IV de la FIG. 2, faite au niveau du manchon en carbone sous une forme pyrolysé, polymérisée, seule ou en association.
La FIG. 5 est une vue en coupe montrant l'implantation d'un cathéter, selon le premier mode de réalisation, sur un site de ponction dans un organisme vivant au travers de l'épithélium avec, en médaillon, un détail à plus grande échelle centré sur le manchon en manchon en carbone sous une forme pyrolysée, polymérisée, seule ou en association.
La FIG. 6 est une vue en perspective et en coupe partielle illustrant, outre l'implantation dans le site de la FIG. 5, le branchement sur le cathéter, d'un dispositif de perfusion (en pointillés).
La FIG. 7 est une vue en perspective montrant un cathéter à manchon en carbone de type pyrolysé, de type polymérisé, seul ou en association selon un deuxième mode de réalisation.
La FIG. 8 est une vue en coupe longitudinale du cathéter de la FIG. 7 avec, en médaillon, des détails à plus grande échelle centrés, en haut, sur le manchon et en bas, sur l'extrémité distale du cathéter.
La FIG. 9 est une vue en coupe montrant l'implantation d'un cathéter, selon le deuxième mode de réalisation, sur un site de ponction dans un organisme vivant au travers de l'épithélium avec, en médaillon, un détail à plus grande échelle centrée sur le manchon en de type pyrolysé, de type polymérisé, seul ou en association.
La FIG. 10 est une vue en perspective et en coupe partielle illustrant, outre l'implantation sur le site de la FIG. 9, le branchement, sur le cathéter, d'une seringue (en pointillés).

On a représenté sur les dessins un cathéter 1. Ce cathéter 1 est apte à (et conçu pour) être inséré dans un organisme 2 vivant (typiquement le corps humain mais il pourrait s'agir d'un animal) par voie percutanée, sur un site 3 de ponction.

La fonction du cathéter 1 est de fournir une voie d'accès par laquelle peut passer ou transiter :
- Un fluide à injecter dans l'organisme, par ex. un soluté, un médicament intraveineux, du sang (naturel ou artificiel) ou encore un produit de contraste ;
- Un fluide à prélever de l'organisme, par ex. du sang ;
- Un dispositif d'injection (par ex. l'aiguille d'une seringue) ;
- Un dispositif de ponction (par ex. un mandrin) ;
- Un autre objet filaire, tel qu'un instrument d'exploration (typiquement une fibre optique), de mesure (sonde), de guidage (lors d'une méthode de type Seldinger) ou de chirurgie (instrument), ou encore un fil conducteur de l'électricité (par ex. pour l'alimentation d'un implant motorisé ou instrumenté, transfert de signal électrique ou informatique).

Le cathéter 1 comprend en premier lieu, à une extrémité 4 proximale, un embout 5 destiné à être positionné à l'extérieur de l'organisme 2.

Le cathéter 1 comprend, en deuxième lieu, une canule 6 qui s'étend à partir de l'embout 5 jusqu'à une extrémité 7 distale destinée à être positionnée à l'intérieur de l'organisme 2.

La canule 6 présente une face 8 interne qui définit un conduit 9 interne, et une face opposée 10 externe, au contact de l'organisme lors de l'introduction.

L'extrémité 7 distale est de préférence effilée, de sorte à faciliter l'insertion de la canule 6 dans l'organisme 2.

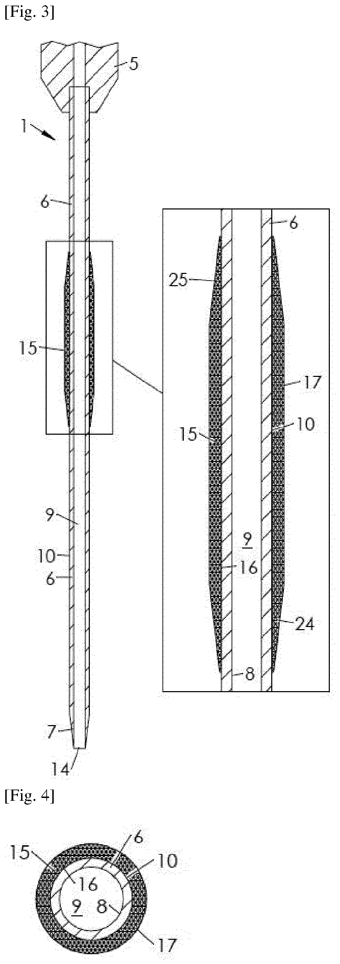

Selon un mode de réalisation, la canule 6 présente en section un contour circulaire (FIG. 4). En variante, cette section pourrait être non circulaire, notamment ovale, polygonale. Dans ce dernier cas, les faces du polygone pourraient être planes, concaves ou convexes dans tout plan de section transversale.

La canule 6 peut comporter sur tout ou partie de sa face 10 externe un relief pour un meilleur ancrage dans la peau, par exemple un filetage pour l'ancrer ou l'insérer en vissant.

Le conduit 9 interne est avantageusement à section circulaire.

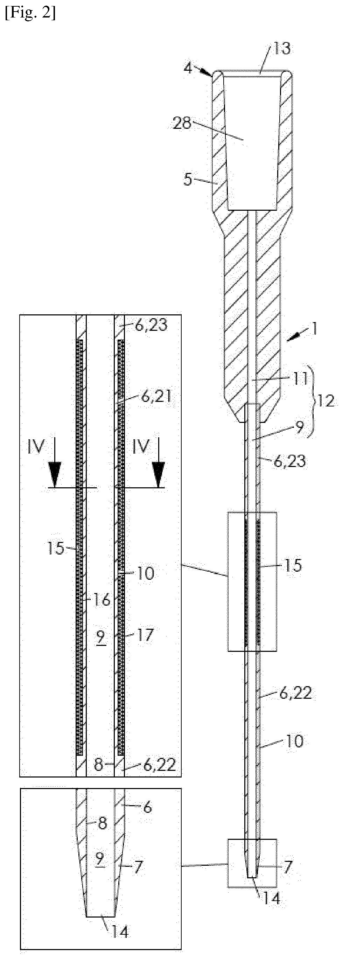

Comme on le voit bien sur les dessins, et notamment sur les FIG. 2 et FIG. 3, l'embout 5 présente un alésage 11 interne qui s'étend dans le prolongement du conduit 9 interne de la canule 6. Ensemble, l'alésage 11 et le conduit 9 forment dans le cathéter 1 un canal 12 qui fournit la voie d'accès précitée, et qui débouche de part et d'autre du cathéter 1 :
- D'une part, du côté de l'extrémité 4 proximale, à une embouchure 13 proximale.
- D'autre part, du côté de l'extrémité 7 distale, à une embouchure 14 distale.

La canule 6 au moins est réalisée dans un matériau biocompatible, par exemple dans un polymère thermoplastique lorsqu'elle n'est pas réalisée intégralement en carbone de type pyrolysé, de type polymérisé, seul ou en association.

Parmi les matériaux envisageables pour la réalisation de la canule 6, citons aussi le polyéthylène, le polypropylène, le silicone, le polyuréthane, le polytétrafluoroéthylène (PTFE).

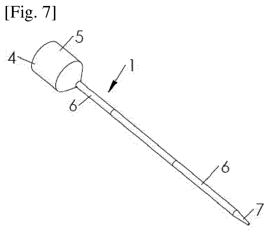

L'embout 5 et la canule 6 peuvent être réalisés sous forme d'un ensemble monobloc (FIG. 7).

En variante toutefois, l'embout 5 et la canule 6 peuvent être deux pièces distinctes assemblées (FIG. 2). Ainsi, l'embout 5 peut être réalisé dans un matériau relativement rigide, tandis que la canule 6 peut être réalisée dans un matériau comparativement plus souple.

Le cathéter 1 comprend en outre un manchon 15 réalisé dans un matériau en carbone de type pyrolysé, de type polymérisé, seul ou en association, qui forme ou recouvre toute ou partie de la face 10 externe de la canule 6.

Le manchon 15 se présente globalement sous forme d'un cylindre dont une face 16 interne épouse la face 10 externe de la canule 6 sur une partie au moins de sa longueur, et dont une face 17 externe, opposée, est destinée à venir au niveau du point d'introduction en contact de l'organisme 2 dans lequel le cathéter 1 est destiné à être inséré. Le manchon peut aussi constituer la totalité de l'épaisseur de la canule sur toute ou partie de sa longueur.

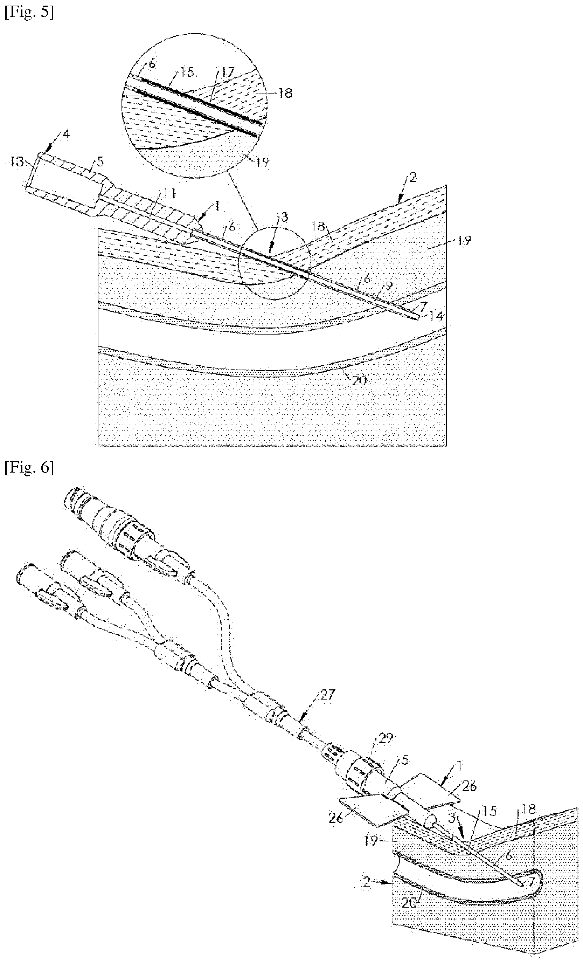

Comme illustré sur les FIG. 5, FIG. 6, FIG. 9 et FIG. 10, l'organisme 2, vu en coupe, présente (de manière simplifiée), au site de ponction :
- Une couche 18 superficielle, l'épiderme, formée de cellules juxtaposées kératinisées, et qui définit la face superficielle de l'organisme 2, par laquelle celui- ci est en contact avec l'air ambiant ; il s'agit d'un épithélium ;
- Dans le cas de la peau, une couche 19 sous-jacente, faite du derme et de l'hypoderme, qui repose sur des tissus conjonctifs ;
- Un réseau 20 vasculaire, au moins partiellement inclus dans les tissus 19 sous-jacents.

Le manchon 15 est destiné, au niveau de la peau, à traverser au moins partiellement l'épiderme 18, et le cas échéant une partie de la couche 19 sous-jacente (derme, hypoderme) pouvant aller plus profondément et traverser alors des organes jusqu'à la destination qui lui est assignée.

La face 17 externe du manchon 15 est destinée à venir au contact au moins de l'épiderme 18 lors de la pose au niveau de la peau.

Selon un mode préféré de réalisation, la face 17 externe du manchon 15 s'étend au moins partiellement dans le prolongement (ou dans la continuité) de la face 10 externe de la canule 9.

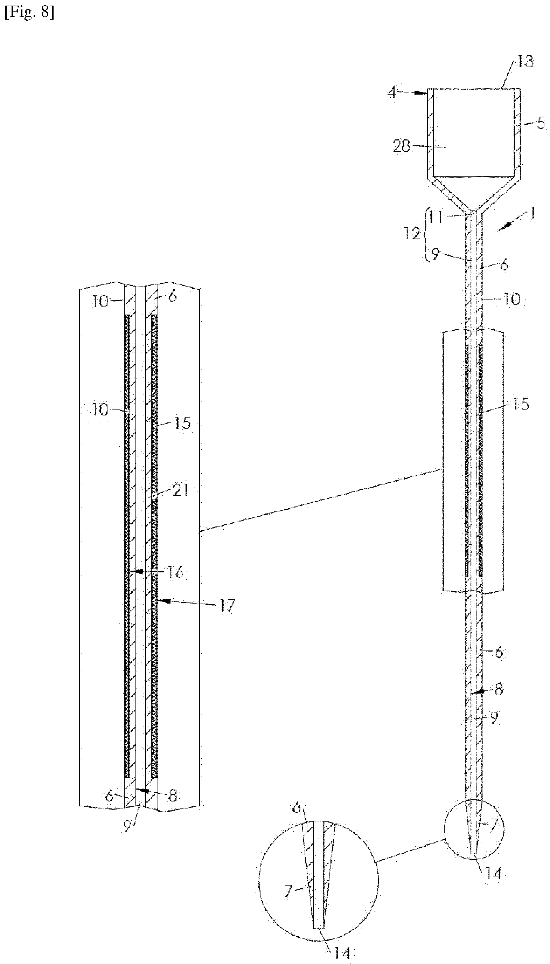

Dans les modes de réalisation illustrés sur les FIG. 2 et FIG. 8, la canule 6 présente, sur une partie de sa longueur, une restriction 21 externe locale. Cette restriction 21 est une zone rétrécie par l'extérieur ; dans le cas où la canule 6 présente en section transversale un contour circulaire, il s'agit d'une zone de moindre diamètre. On voit sur la FIG. 2 que le manchon 15 s'étend dans la restriction 21. Sa face 17 externe s'étend avantageusement dans le prolongement (autrement dit, dans la continuité) de la face 10 externe de la canule 6.

Pour obtenir une telle configuration, la canule 6 peut être recouverte d'un manchon en carbone de type pyrolysé, de type polymérisé, seul ou en association, ou fabriquée par surmoulage : le manchon 15 déjà usiné ou moulé dans sa forme finale est positionné dans un moule d'injection plastique, puis le matériau de la canule 6 est injecté sous forme pâteuse ou liquide pour venir épouser le manchon 15. Le carbone peut être pulvérisé, éventuellement à chaud, pour être déposé sur la face externe 10 de la canule 6. Les fibres de carbones peuvent aussi être appliquées par thermocollage, ou déposées sur la surface de la canule et rendues adhérente par l'application de polymères de carbone par exemple.

Comme on le voit sur la FIG. 2, la matière de la canule 6 se trouve dans le manchon 15 et de part et d'autre de celui-ci, dans la direction axiale, de sorte à former une section 22 aval (du côté de l'extrémité 7 distale) et une section 23 amont (du côté de l'embout 5) de même diamètre externe que le manchon 15.

Dans une première variante, et dans l'hypothèse où le matériau de la canule 6 serait suffisamment souple, la canule 6 peut être fabriquée indépendamment du manchon 15, puis ce dernier est enfilé sur la canule 6 jusqu'à venir se loger dans la restriction 21.

Dans une deuxième variante, illustrée sur la FIG. 3, le manchon 15 peut former, par rapport à au moins une partie de la canule 6, une surépaisseur.

Le manchon 15 est de préférence effilé à une extrémité 24 située du côté de l'extrémité 7 distale de la canule 6 (en d'autres termes, à l'opposé de l'épaulement 23) de manière à faciliter son insertion. Le cas échéant, et comme illustré sur la FIG. 3, le manchon 15 est également effilé à une extrémité 25 située du côté du manchon 5.

Pour assurer un bon maintien du manchon 15 sur la canule 6, ainsi qu'une bonne étanchéité à leur interface, un promoteur d'adhérence (notamment une colle, de préférence biocompatible) peut être interposé entre eux.

L'embout 5 peut adopter diverses configurations, selon l'usage auquel est destiné le cathéter 1.

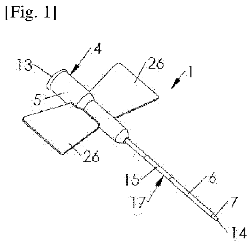

Ainsi, dans l'exemple des FIG. 1 à FIG. 6, l'embout 5 est pourvu d'ailettes 26 destinées à faciliter la prise en main du cathéter 1 pour une insertion directe de la canule 6 dans l'organisme 2, l'extrémité 7 distale effilée assurant la perforation de l'épiderme 18 sans l'aide d'un instrument supplémentaire tel qu'une aiguille.

L'embout peut aussi servir de prise pour être connecté à une source électrique ou un matériel informatique.

Un tel cathéter 1 peut être employé en perfusion, cf. la FIG. 6 sur laquelle on a illustré le branchement, sur l'embout 5, d'un dispositif 27 de perfusion (en pointillés).

À cet effet, l'embout 5 est avantageusement muni d'un logement 28 en creux, qui peut être tronconique, et le cas échéant pourvu d'un filetage interne, pour accueillir un raccord 29 prévu par ex. à l'extrémité du dispositif 27 de perfusion.

Dans l'exemple des FIG. 7 à FIG. 10, le cathéter 1 est conformé pour permettre notamment le passage d'une aiguille 30 d'une seringue 31 pour l'injection d'un fluide dans l'organisme 2 ou, au contraire, le prélèvement d'un fluide organique.

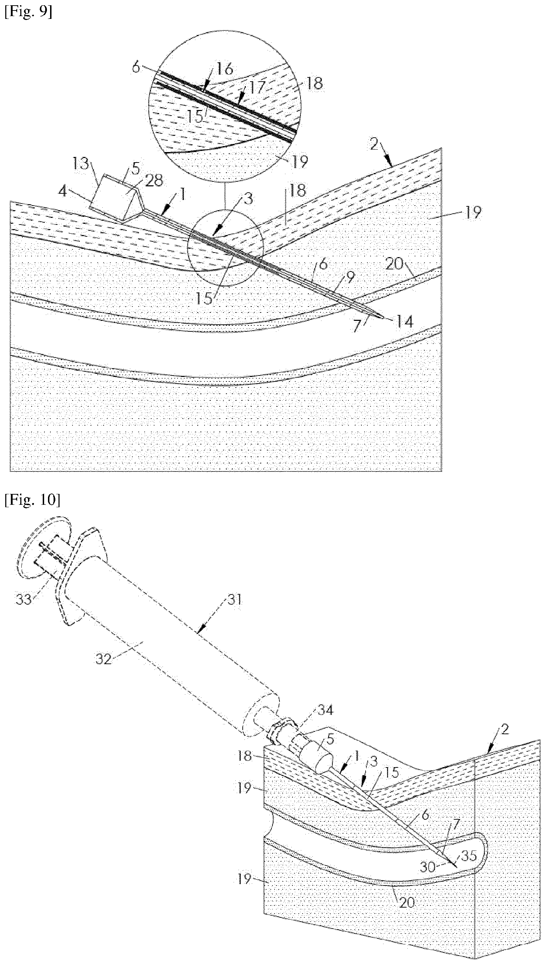

Une seringue 31 typique est illustrée sur la FIG. 10 ; elle comprend un corps 32 tubulaire dans lequel est monté un piston 33, et une aiguille 30 fixée sur le corps 32 par l'intermédiaire d'un raccord 34.

Dans cet exemple, et comme illustré sur la FIG. 8, l'embout 5 est aussi muni d'un logement 28 en creux dont le raccord 34 est complémentaire, celui-ci venant s'y emboîter.

L'insertion du cathéter 1 dans l'organisme 2 (et plus précisément dans le site 3 de ponction) est avantageusement effectuée en biais, mais elle peut être effectuée de manière perpendiculaire.

Les insertions représentées sur les FIG. 5, FIG. 6, FIG. 9 et FIG. 10 sont de type intra- vasculaire, c'est-à-dire qu'elles sont destinées à créer une voie d'accès entre l'extérieur de l'organisme 2 et le réseau 20 vasculaire (dont on a représenté partiellement une voie veineuse dans laquelle débouche l'extrémité 7 distale de la canule 6), mais ces représentations sont illustratives et non limitatives.

Dans les cas où le cathéter 1 doit être inséré dans l'organisme 2 sans recourir à un instrument tel qu'une aiguille, l'insertion est facilitée par le caractère effilé de l'extrémité 7 distale, qui peut ainsi perforer l'épiderme 18.

Dans les cas où le cathéter 1 doit être inséré dans l'organisme 2 en même temps que l'instrument (typiquement l'aiguille 30 illustrée sur la FIG. 10), l'insertion du cathéter 1 est concomitante à celle de l'instrument. Dans le cas de la seringue 31, l'aiguille 30 est d'abord enfilée dans le canal 12 du cathéter 1 de sorte à ce qu'une extrémité 35 effilée de l'aiguille 30 dépasse de l'extrémité 7 distale de la canule 6, et l'insertion du cathéter 1 dans l'organisme est favorisée par la perforation de l'épiderme 18 initiée par l'aiguille 30.

Dans tous les cas, il convient de positionner le manchon 15 au niveau de la couche superficielle de l'épithélium 18.

En effet, il a pu être démontré qu'un épithélium (en l'espèce l'épiderme 18, ou une muqueuse) non seulement ne rejette pas le manchon 15 en carbone de type pyrolysé, de type polymérisé, seul ou en association, mais vient cicatriser sur lui en l'intégrant, c'est-à-dire en adhérant sur sa face 17 externe au niveau cellulaire.

En d'autres termes, la face 17 externe du manchon 15 en carbone de type pyrolysé, de type polymérisé, seul ou en association est complètement intégrée à l'épithélium 18 sans interposition fibreuse ni formation d'un espace péri-prothétique entre eux, formant ainsi une barrière étanche y compris à la propagation bactérienne.

Ainsi, dès lors que le manchon 15 est correctement positionné au niveau de l'épithélium 18, il est rapidement intégré par celui-ci comme s'il s'agissait d'une partie de lui-même, sans réaction à corps étranger.

Après l'insertion du cathéter 1, le manchon 15 se trouve recouvert intimement par les cellules de l'épithélium 18 avec une adhésion entre eux au niveau cellulaire.

Passé un temps de cicatrisation, l'interface entre le manchon 15 et l'épiderme 18 apparaît dénuée de foyer inflammatoire ou infectieux ; elle se révèle former, au point d'entrée de la canule 6, une barrière aux bactéries et champignons.

La liaison ainsi réalisée entre le manchon 15 (et donc le cathéter 1) d'une part, et l'épiderme 18 (ou tout autre épithélium) d'autre part, est qualifié d'épithélio-intégration, par analogie avec l'ostéo-intégration de certains matériaux (notamment en titane) ou de certaines prothèses, qui ont la particularité de réaliser une liaison similaire avec le tissu osseux.

Quant à la capacité d'un matériau (en l'occurrence du carbone de type pyrolysé, de type polymérisé, seul ou en association) ou d'un composant (en l'espèce le manchon 15 en carbone) à lier un contact intime (sans rejet comme corps étranger, sans foyer inflammatoire, sans foyer infectieux) avec les cellules de l'épiderme 18 (ou de tout autre épithélium), elle est qualifiée d'épithélio-intégrabilité. Il se forme une adhérence qui peut servir d'ancrage.

On peut observer que l'ostéo-intégrabilité d'un matériau n'implique pas son épithélio-intégrabilité. Ainsi, le titane, ostéo-intégrable, n'est pas épithélio-intégrable : les tissus de l'épithélium ne le rejettent pas forcément, mais ne nouent pas avec ce matériau un contact cellulaire intime formant une barrière étanche aux bactéries et champignons comme c'est le cas pour le carbone.

L'épithélio-intégrabilité du carbone de type pyrolysé, de type polymérisé, seul ou en association dont est fait le manchon 15 du cathéter 1, est déterminante car elle renverse la doctrine médicale d'usage des cathéters. En effet :
- Le cathéter 15 à manchon 15 en carbone devient un dispositif pérenne ;
- Il n'est pas, ou peu, sujet à la prolifération de bactéries et champignons depuis le point d'insertion, car le point d'entrée est étanche à leur migration, même lorsque la surface externe de l'épiderme 18 (ou de tout autre épithélium) est elle-même colonisée ;
- Après la pose, la désinfection de l'interface entre le manchon 15 et l'épiderme 18 au point d'entrée du cathéter 1 n'est plus aussi impérative, ou l'est avec une moindre fréquence et à titre prophylactique ;
- Le cathéter 1 peut demeurer en insertion organique sur le site 3 de ponction pendant une longue durée (plusieurs jours, plusieurs semaines, voire plusieurs mois ou années) avec un risque infectieux moindre.

Outre la diminution du risque infectieux, il en résulte des avantages en termes d'usage :
- L'adhésion cellulaire de l'épiderme 18 (ou de tout autre épithélium) sur le manchon 15 en carbone de type pyrolysé, de type polymérisé, seul ou en association, lors de son intégration induit un bon ancrage du cathéter 1 dans l'organisme 2, ce qui diminue le risque de déplacement ;
- Le confort du patient équipé du cathéter 1 peut être considérablement amélioré ;
- Les pansements de maintien et/ou de protection couvrant le point d'entrée peuvent être allégés voire même supprimés.

On aura bien entendu avantage à maintenir l'asepsie de l'embout 5 et de sa connexion, afin d'éviter, lors des manipulations précitées, l'introduction de micro- organismes dans l'organisme par le canal 12 ; on notera cependant qu'une telle précaution vaut également pour les cathéters ordinaires. De même, lorsque le manchon 15 ne constitue pas la totalité de l'épaisseur de la canule 6, le contact entre la surface interne du manchon et la face externe 10 de la canule doit être étanche.

## Revendications

1. Cathéter (**1**) insérable dans un organisme (**2**) vivant par voie percutanée pour l'administration ou le prélèvement d'un fluide ou l'introduction d'un instrument ou d'un objet filaire, pour la réalisation de gestes médicaux ou chirurgicaux, ou pour le passage d'un signal électrique, ce cathéter comprenant :
- A une extrémité (**4**) proximale, un embout (**5**) destiné à être positionné à l'extérieur de l'organisme (**2**) ;
- Une canule (**6**) qui s'étend à partir de l'embout (**5**) jusqu'à une extrémité (**7**) distale destinée à être positionnée à l'intérieur de l'organisme (**2**), cette canule (**6**) ayant une face (**8**) interne définissant un conduit (**9**) interne, et une face (**10**) externe ;
- Un manchon (15) servant à minimiser le risque d'infection ;
**caractérisé en ce que** le manchon (**15**) est réalisé dans un matériau composé à 95% au moins de carbone, qu'il soit de type pyrolysé sous forme de matrice, sous forme pulvérisée, sous forme filaire ou sous forme composite filaire dans une matrice, qu'il soit de type polymérisé comme le carbone amorphe hydrogéné (par exemple le poly-hydridocarbyne) comme le Diamon-like carbone ou le polymer-like carbone, ou qu'il soit par association des types pyrolysés et polymérisés sous leurs différentes formes, qui compose ou qui recouvre la face (**10**) externe de la canule (**6**).

2. Cathéter (**1**) selon la revendication 1, **caractérisé en ce que** le manchon (**15**) recouvre une partie au moins de la longueur de la canule (**6**).

3. Cathéter (**1**) selon la revendication 1, **caractérisé en ce que** la canule (**6**) présente, sur une partie de sa longueur, une restriction (**21**) externe locale, dans laquelle s'étend le manchon (**15**).

4. Cathéter (**1**) selon la revendication 3, **caractérisé en ce que** le manchon (**15**) présente une face (**17**) externe qui s'étend dans le prolongement de la face (**10**) externe de la canule (**6**).

5. Cathéter (**1**) selon la revendication 3 ou la revendication 4, **caractérisé en ce que** la canule (**6**) est surmoulée à l'intérieur du manchon (**15**).

6. Cathéter (**1**) selon la revendication 1 ou la revendication 2, **caractérisé en ce que** le manchon (**15**) forme, par rapport à au moins une partie de la canule (**6**), une surépaisseur.

7. Cathéter (**1**) selon la revendication 6, **caractérisé en ce que** la canule (**6**) présente une section (**22**) de moindre diamètre sur laquelle est enfilé le manchon (**15**).

8. Cathéter (**1**) selon la revendication 7, **caractérisé en ce que** le manchon (**15**) est effilé au moins à une extrémité (**35**) située du côté de l'extrémité (**7**) distale de la canule (**6**).

9. Cathéter (**1**) selon l'une des revendications précédentes, **caractérisé en ce que** la canule (**6**) est réalisée dans un polymère thermoplastique biocompatible.

## Patentansprüche

1. Katheter (1), der perkutan in einen lebenden Organismus (2) eingeführt werden kann, um eine Flüssigkeit zu verabreichen oder zu entnehmen oder ein Instrument oder einen verdrahteten Gegenstand einzuführen, um medizinische oder chirurgische Eingriffe durchzuführen oder um ein elektrisches Signal zu leiten, dieser Katheter umfassend :
- an einem proximalen Ende (4), eine Spitze (5), die außerhalb des Körpers positioniert werden soll (2)
- eine Kanüle (6), die sich von der Spitze (5) bis zu einem distalen Ende (7) erstreckt und dazu bestimmt ist, innerhalb des Organismus (2) positioniert zu werden, wobei diese Kanüle (6) eine Innenseite, (8) die einen Innenkanal (9) bildet, und eine Außenseite (10) hat,
- eine Buchse (15) die dazu dient, das Infektionsrisiko zu minimieren, **dadurch gekennzeichnet ist, dass** die Buchse (15) in einem Material ausgeführt ist, das aus mindestens 95% Kohlenstoff besteht, sei es entweder als pyrolysierter Art in Form einer Matrix, in pulverisierter Form, in Drahtform oder in Drahtverbundform in einer Matrix, oder als polymerisierter Art wie hydrierter amorpher Kohlenstoff (zum Beispiel Polyhydridocarbyl), diamonartiger Kohlenstoff oder polymerartiger Kohlenstoff oder als Verbindung der pyrolysierten und polymerisierten Arten in ihren verschiedenen Formen, die die Außenseite (10) der Kanüle (6) bilden oder bedecken.

2. Katheter (1) nach Anspruch 1, **dadurch gekennzeichnet, dass** die Buchse (15) mindestens einen Teil der Länge der Kanüle (6) bedeckt.

3. Katheter (1) nach Anspruch 1, **dadurch gekennzeichnet, dass** die Kanüle (6) über einen Teil ihrer Länge eine lokale äußere Einschränkung (21) aufweist, in der sich die Buchse erstreckt.

4. Katheter (1) nach Anspruch 3, **dadurch gekennzeichnet, dass** die Buchse (15) eine Außenseite (17) aufweist, die sich in die Verlängerung der Außenseite (10) der Kanüle (6) erstreckt.

5. Katheter (1) nach Anspruch 3 oder Anspruch 4, **dadurch gekennzeichnet, dass** die Kanüle (6) im Inneren der Buchse aufgeformt ist (15).

6. Katheter (1) nach Anspruch 1 oder Anspruch 2, **dadurch gekennzeichnet, dass** die Buchse (15) relativ zu mindestens einem Teil der Kanüle (6) eine Verdickung bildet.

7. Katheter (1) nach Anspruch 6, **dadurch gekennzeichnet, dass** die Kanüle (6) einen Abschnitt (22) mit kleinerem Durchmesser aufweist, auf den die Buchse (15) aufgeschraubt ist.

8. Katheter (1) nach Anspruch 7, **dadurch gekennzeichnet, dass** die Buchse (15) mindestens an einem Ende (35) verjüngt ist, das sich an der Seite des (7) distalen Endes der Kanüle (6) befindet.

9. Katheter (1) nach einem der vorhergehenden Ansprüche, **dadurch gekennzeichnet, dass** die Kanüle (6) aus einem biokompatiblen thermoplastischen Polymer besteht.

## Claims

1. Catheter (1) insertable into a living organism (2) percutaneously for the administration or withdrawal of a fluid or the introduction of an instrument or a wired object, in order to perform medical or surgical procedures, or for the passage of an electrical signal, this catheter comprising:
- at a proximal end (4), a tip (5) intended to be positioned outside the body (2),
- a cannula (6) which extends from the tip (5) to a distal end (7) intended to be positioned inside the organism (2), this cannula (6) having an internal face (8) defining an internal duct (9), and an external face (10),
- a sleeve (15) serving to minimize the risk of infection, **characterised in that** the sleeve (15) is made of a material composed of at least 95% carbon, whether of pyrolyzed form as a matrix, pulverized form, in wire form or in wire and composite form in a matrix, whether it is of the polymerized type such as hydrogenated amorphous carbon (for example poly-hydridocarbyl) such as Diamon-like carbon or polymer-like carbon, or whether it is by association of the pyrolyzed and polymerized types in their different forms, which composes or covers the outer face (10) of the cannula (6).

2. Catheter (1) according to claim 1, **characterised in that** the sleeve (15) covers at least part of the length of the cannula (6).

3. Catheter (1) according to claim 1, **characterised in that** the cannula (6) has, over part of its length, a local external restriction (21), in which the sleeve extends (15).

4. Catheter (1) according to claim 3, **characterised in that** the sleeve (15) has an outer face (17) which extends in the continuation of the outer face (10) of the cannula (6).

5. Catheter (1) according to claim 3 or claim 4, **characterised in that** the cannula (6) is overmolded inside the sleeve (15).

6. Catheter (1) according to claim 1 or claim 2, **characterised in that** the sleeve (15) forms an extra thickness, relative to at least part of the cannula (6).

7. Catheter (1) according to claim 6, **characterised in that** the cannula (6) has a section (22) of smaller diameter on which the sleeve is threaded (15).

8. Catheter (1) according to claim 7, **characterised in that** the sleeve (15) is tapered at least at one end (35) located at the distal end (7) of the cannula (6).

9. Catheter (1) according to one of the preceding claims, **characterised in that** the cannula (6) is made of a biocompatible thermoplastic polymer.
